(19) Europäisches Patentamt

European Patent Office

Office européen des brevets

(11) **EP 0 850 416 B1**

(12) **EUROPÄISCHE PATENTSCHRIFT**

(45) Veröffentlichungstag und Bekanntmachung des Hinweises auf die Patenterteilung:
**01.08.2001 Patentblatt 2001/31**

(51) Int Cl.[7]: **G01N 33/53**

(86) Internationale Anmeldenummer:
**PCT/DE96/01600**

(21) Anmeldenummer: **96943847.2**

(22) Anmeldetag: **23.08.1996**

(87) Internationale Veröffentlichungsnummer:
**WO 97/08930 (13.03.1997 Gazette 1997/12)**

(54) **VERFAHREN ZUR ZWEIDIMENSIONALEN BESTIMMUNG VON PROBEN IN IMMUNOASSAYS**

METHOD FOR TWO-DIMENSIONAL MEASUREMENT OF SAMPLES IN IMMUNOASSAYS

PROCEDE DE DETECTION BIDIMENSIONNELLE D'ECHANTILLONS DANS DES IMMUNODOSAGES

(84) Benannte Vertragsstaaten:
**BE CH FR GB IE IT LI NL SE**

(30) Priorität: **24.08.1995 DE 19532655**

(43) Veröffentlichungstag der Anmeldung:
**01.07.1998 Patentblatt 1998/27**

(73) Patentinhaber: **STROHNER, Pavel**
**13187 Berlin (DE)**

(72) Erfinder: **STROHNER, Pavel**
**13187 Berlin (DE)**

(74) Vertreter: **Baumbach, Friedrich**
**BioTeZ Berlin-Buch GmbH**
**Patentstelle**
**Robert-Rössle-Strasse 10**
**13125 Berlin (DE)**

(56) Entgegenhaltungen:
**WO-A-93/10451**

## Beschreibung

Die Erfindung bezieht sich auf ein Verfahren zur richtigen immunchemischen Bestimmung eines Analyten (Antigen) in Proben, in denen Fremdstoffe enthalten sind, die die Messung des Analyten mittels Immunoassay-Standardkurve stören.

Anwendungsgebiete der Erfindung sind die medizinische und veterinärmedizinische Diagnostik, die Nahrungs- und Umweltanalytik, die immunologische und pharmakologische Forschung.

Bisher bekannt ist folgender Stand der Technik:

Der Immunoassay ist eine sehr verbreitete Technik zur Bestimmung unbekannter Konzentrationen analytisch oder diagnostisch relevanter Stoffe (Analyt) in Probenmaterial wie Serum, Plasma, Gewebsproben, Rückstandspräparationen und ähnlichem.

Die Immunoassays (Radioimmunoassays(RIA),Enzymimmunoassays (EIA), Lumineszenzimmunoassays (LIA) und damit verwandte Assayvarianten) basieren darauf, daß eine Analytkonzentration als Standard vorgegeben wird und für diese nach Reaktion mit einem Antikörper unter Einbeziehung einer markierten Verbindung (eines markierten Antigens oder Antikörpers) die Response des gebundenen markierten Antigens oder Antikörpers (Impulsrate, Extinktion, Relative Light Units) bestimmt wird. Der Zusammenhang zwischen Response und Analytkonzentration des Standards wird in Form einer Standardkurve mittels mathematischer Eichfunktion oder graphisch beschrieben.

Die Analytkonzentration unbekannter Proben wird unter Verwendung der nach der Analytkonzentration umgeformten Eichfunktion aus der Response der unbekannten Probe errechnet oder wird an der graphisch erstellten Standardkurve abgelesen. Bei der Eichung mit Hilfe einer Standardkurve werden gewöhnlich zur Vermeidung der Beeinflussung der Messung durch Fremdstoffe besondere Vorbehandlungen der Proben (z.B. Extraktionen, Zugabe von Zusatzstoffen) oder der Assaykomponenten (Einbringung der Standards in 0-Seren, Proteinzusätze u.a.m.) vorgenommen. Mit Wiederfindexperimenten wird die Richtigkeit der Bestimmungen an den so modifizierten Assaykomponenten oder Proben geprüft.

Softwarepakete an den Meßgeräten (Gamma-Counter, Extinktions-Reader, Lumineszenz-Meßgeräte), die die Immunoassay-Auswertung übernehmen, sind Standardausstattung von Immunoassay-Forschungs- und Routinelabors geworden. Darüberhinaus sind Immunoassay-Laborautomaten verschiedenster Hersteller auf dem Markt, die sowohl die Abarbeitung eines Immunoassays als auch seine Messung und Auswertung in einem Automaten vereinen. Allen diesen Auswerte-Software-Paketen und Labor-Automaten liegt o.g. Eichverfahren zugrunde, wenn auch heute, auf Grund der besseren Beherrschung der Assaytechnik, gespeicherte Eichfunktionen für mehrere Assayläufe oder auf zwei Standardkonzentrationen reduzierte Eichgeraden üblich geworden sind.

Literatur

**Rodbard D., Ratford P.L., Cooper J. and Ross G.T.** (1968).
Statistical quality control of radioimmunoassays. J. Clin. Endocrinol. Metab. 29, 352.

**Azimzadeh A., Pellequer J.L. and Van Regenmortel M.H.V.** (1992). Operational aspects of antibody affinity constants measured by liquid-phase and solid-phase assays. J. Molecular Recognition. 5, 9.

**Goldberg M.E. and Djavadi-Ohaniance L.** (1993). Methods for measurement of antibody/antigen affinity based on ELISA and RIA.
Current Opinion in Immunology 5, 278.

Trotz dieser Rationalisierungen und Erkenntnisfortschritte bestehen noch immer erhebliche Schwierigkeiten bei der systematischen Beherrschung der durch kreuzreaktive Stoffe und Matrixeffekte verursachten Fehlbestimmungen im Probenmaterial.

WO-A-93/10 451 betrifft ein Verfahren zur Schnell-Optimierung und Qualitätskontrolle von Immuntest verfahren sowie eine Vorrichtung zur Durchführung des Verfahrens. Das Verfahren ist dadurch gekennzeichnet daß die Bindungsraten eines bestimmten Antigen-Antikorperkorperkomplexes, gegebenenfalls unter zufügung eines oder mehrerer Stoffe oder nach Verdünnung zwei- oder mehr dimensional bestimmt werden.

Anhand der so erhaltenen Eichdaten erfolgt die mathematische Auswertung für die Anpassung und Optimierung des Assays.

Der Erfindung liegt die Aufgabe zugrunde, ein Verfahren zur richtigen immunchemischen Bestimmung eines Analyten zu entwickeln, bei dem die in den Untersuchungsproben gewöhnlich vorhandenen Fremdstoffe in ihrer Wirkung auf die Probenbestimmung erfaßt und berücksichtigt werden.

Das Verfahren soll so gestaltet werden, daß keine gesonderten Vorbehandlungen, wie Extraktionen, Zugabe von Zusatzstoffen o.ä. am als Flüssigkeit vorliegenden Standard- oder Probenmaterial erforderlich sind.

Die Erfindung wird gemäß Anspruch 1 realisiert, Anspruch 2 beansprucht eine Vorzugsvariante.

Kernstück der Erfindung ist die zweidimensionale Eichung des zur Anwendung vorgesehenen Immunoassays, wobei neben der Standardkonzentration des Antigens als weitere unabhängige Variable die Antikörperkonzentration herangezogen wird. Bei der nachfolgenden Bestimmung eines Analyten in unbekannten Proben wird bei mehreren Antikörperkonzentrationen gemessen, und zwar genau bei denen auch die Eichung erfolgte. Anschließend erfolgt ein Vergleich mit den Eichkurven, wobei man die systematische Änderung der in der Probe ermittelten Antigenkonzentration in Abhängigkeit von der Antikörperkonzentration für die Messung ausnutzt.

Die Erfindung beruht auf der Erkenntnis, daß bei einer gestörten Probe sich bei unterschiedlicher Antikörperkonzentration unterschiedliche Antigenkonzentrationen ergeben und diese Störung einer einfachen Gesetzmäßigkeit folgt.

Gegenstand der Erfindung sind auch alle Vorrichtungen, mit denen das erfindungsmäßige Verfahren durchgeführt wird. Eine solche Vorrichtung kann beispielsweise ein Immunoassay-Reagenziensatz sein, der statt bisher mit einer mit mehreren Konzentrationen eines Antikörpers arbeitet oder ein Laborautomat, bei dem zur Eichung und zur Probenbestimmung mehrere Konzentrationen eines Antikörpers herangezogen werden.

Die Erfindung wird im folgenden näher beschrieben:

1. Die Eichung erfolgt zweidimensional, es wird der Zusammenhang zwischen Standard-Konzentration als unabhängige Variable und Response als abhängige Variable um die Antikörperkonzentration als zusätzliche unabhängige Variable erweitert. Zweidimensional bedeutet in diesem Zusammenhang, daß anstelle einer (der Standardkonzentration) zwei unabhängige Variable (Standard- und Antikörperkonzentration) zur Eichung herangezogen werden.

Im praktischen Assayablauf bedeutet das, daß die gleiche Standardkurve bei 2 bis 7 verschiedenen Antikörperkonzentrationen aufgenommen wird. Die Eichfunktion für diesen mehrdimensionalen Zusammenhang muß so gewählt werden, daß die Rekalkulation der Standardkonzentrationen mittels Eichfunktion keine signifikanten Abweichungen von den vorgegebenen Standardkonzentrationen zeigt, d.h. auch bei unterschiedlicher Antikörperkonzentration muß bei gleicher vorgegebenen Standardkonzentration die rekalkulierte Standardkonzentration gleich bleiben. Die Parameter der Eichfunktion können mit Hilfe der linearen und nichtlinearen Regression aus den Konzentrationswerten der unabhängigen Variablen und aus den Responsewerten ermittelt werden. Mögliche verwendungsfähige Eichfunktionen sind in den Beispielen aufgeführt. Das Eichverfahren ist jedoch nicht auf diese beschränkt. Jede mathematische Funktion, die in der Lage ist, den Zusammenhang zwischen Response einerseits und Analyt- und Antikörperkonzentration andererseits mittels Regression aus den Versuchsdaten richtig wiederzugeben, kann für die zweidimensionale Eichung Verwendung finden. Es können die Eichkurven auch graphisch aufgetragen werden, wobei die unterschiedlichen Eichkurven, die durch die Variation der Antikörperkonzentration entstehen, als Isolinien eingetragen werden können.

2. Unbekannte Proben werden bei mehreren Antikörperkonzentrationen, wie bei der Messung der Standards vorgegeben, im Assay angesetzt und gemessen. Die Konzentrationswerte der unbekannten Proben werden mit Hilfe der durch Regression ermittelten zweidimensionalen Eichfunktion oder auch graphisch bestimmt. Bei einer völlig ungestörten Probe sind die Analytkonzentrationen bei unterschiedlicher Antikörperkonzentration im Rahmen des Versuchsfehlers gleich (ein seltener Fall).

Bei einer gestörten Probe (der Regelfall) ändern sich die Konzentrationswerte der gleichen Probe bei unterschiedlicher Antikörperkonzentration.

Die Störung kann aus zwei verschiedenen Komponenten bestehen,

a) aus einer zum Analyten (Antigen, Hapten) kreuzreaktiven, unbekannten Substanz in den Proben
b) aus einer zum Antikörper kreuzreaktiven, unbekannten Substanz in den Proben

Die Wirkungen dieser beiden Störungen sind grundverschieden.

Eine zum Analyten kreuzreaktive Substanz wirkt sich so aus, daß die ermittelte Analytkonzentration um ein konstanten Betrag von der tatsächlichen Analytkonzentration abweicht.

Eine zum Antikörper kreuzreaktive Substanz bewirkt eine Verfälschung der Analytkonzentration in Abhängigkeit von der Antikörperkonzentration.

3. Die richtige Analytkonzentration kann durch folgendes Vorgehen bestimmt werden:

a) beim kompetitiven Assay (markiertes Antigen als Tracer) Die ermittelten Analytkonzentrationen werden gegen die Antikörperkonzentration graphisch aufgetragen oder es werden mittels linearer Regression unter Nutzung der Funktionen

$$\ln(y) = a + b \cdot x \qquad \text{bzw.} \qquad 1/y = a + b \cdot x$$

(y : Antikörperkonzentration x : Antigenkonzentration a und b: absolutes Glied und Anstieg der linearen Gleichung) die Parameter a und b der linearen Gleichung bestimmt. Welche der beiden Gleichungen Verwendung findet, hängt davon ab, welche der gewählten Gleichungen besser mit den experimentellen Werten übereinstimmt. Andere einfache Funktionen, die die Änderung der Analytkonzentration in Abhängigkeit von der Antikörperkonzentration mit guter Korrelation wiedergeben, können ebenfalls Verwendung finden.

Die richtige Analytkonzentration einer Probe findet man angenähert bei einer möglichst hohen Antikörperkonzentration. Man setzt die höchste Antikörperkonzentration,die experimentell noch sinnvolle Werte ergibt, in die nach x umgeformte Gleichung ein und errechnet x. Von diesem x ist die zum Antigen kreuzreaktive Komponente in den Proben abzuziehen. Die zum Antigen (Analyten) kreuzreaktive Komponente, falls vorhanden, kann am sichersten in Probenmaterial, aus denen der Analyt entfernt wurde, d.h. in sogenannte 0-Proben in gleicher Weise, wie für die Proben angegeben, bestimmt werden.

Alternativ dazu kann die kreuzreaktive Komponente auch in Proben, die bereits das Antigen (den Analyten) enthalten, durch Wiederfindeexperimente ( Aufstockungs- und Verdünnungsexperimente) im zweidimensionalen Ansatz ermittelt werden.

<u>b) beim nichtkompetitiven Assay (markierter Antikörper als Tracer)</u>

Die ermittelten Analytkonzentrationen werden gegen die Antikörperkonzentration graphisch aufgetragen oder es werden mittels linearer Regression unter Nutzung der Funktionen

$$\ln(y) = a + b \cdot x \qquad bzw. \qquad 1/y = a + b \cdot x$$

(y : Antikörperkonzentration x : Antigenkonzentration a und b: absolutes Glied und Anstieg der linearen Gleichung) die Parameter a und b der linearen Gleichung bestimmt. Welche der beiden Gleichungen Verwendung findet, hängt davon ab, welche der gewählten Gleichungen besser mit den experimentellen Werten übereinstimmt. Andere einfache Funktionen, die die Änderung der Analytkonzentration in Abhängigkeit von der Antikörperkonzentration mit guter Korrelation wiedergeben, können ebenfalls Verwendung finden.

Die richtige Analytkonzentration einer Probe findet man angenähert bei einer möglichst niedrigen Antikörperkonzentration, d.h. bei einer Antikörperkonzentration, die noch sinnvoll auswertbare Responsewerte zuläßt.

Man setzt die geringste, experimentell noch verwendbare Antikörperkonzentration in die nach x umgeformte Gleichung ein und errechnet x. Von diesem x ist die zum Antigen kreuzreaktive Komponente in den Proben abzuziehen.

Die zum Antigen (Analyten) kreuzreaktive Komponente, falls vorhanden, kann am sichersten in Probenmaterial, aus denen der Analyt entfernt wurde, d.h. in sogenannte 0-Proben in gleicher Weise, wie für die Proben angegeben, bestimmt werden.

Alternativ dazu kann die kreuzreaktive Komponente auch in Proben, die bereits das Antigen (den Analyten) enthalten, durch Wiederfindeexperimente ( Aufstockungs- und Verdünnungsexperimente) im zweidimensionalen Ansatz ermittelt werden.

4. In der Phase der Entwicklung von Immunoassays sollte die Probenbestimmung bei mehr als 2 verschiedenen Antikörperkonzentrationen erfolgen, um so über mehrere Stützwerte für die Regression 1/y bzw. ln(y) gegen x zu verfügen.

Für die Routineanwendung und bekannten Kurvenverlauf genügen zwei sinnvoll ausgewählte Antikörperkonzentrationen zur Probenbestimmung. Falls die unspezifische Bindung (nsb) sich zwischen Proben und Standards unterscheidet, ist diese in den Proben gesondert zu bestimmen und die Response ist um den Unterschied in der unspezischen Bindung zwischen Probe und Standards zu korrigieren.

Das erfindungsgemäße Verfahren schließt alle Varianten von Immunoassays, wie z.B. RIA, EIA und LIA als auch IRMA (immunoradiometrischer Assay) und seine nicht radioaktiven Varianten als auch die Immuno-PCR, ein.

Das Verfahren ermöglicht die richtige Bestimmung eines Analyten auch in sehr unsauberen Proben. Es ist außerdem ein sehr geeignetes Qualitätskontrollinstrument für bereits etablierte Immunoassays. Die Messung eines Analyten mit einem Immunoassay ist danach nur als richtig anzusehen, wenn in einer Probe bei unterschiedlicher Antikörperkonzentration tatsächlich die gleiche Analytkonzentrationen gemessen wird.

Die der Erfindung zugrunde liegende Entdeckung ist als Basisinnovation auf dem Gebiet der Biodiagnostik zu betrachten, die vielfältige neue Möglichkeiten in der biochemischen und molekularbiologischen Forschung und Diagnostik erschließen wird.

An nachfolgenden Beispielen soll das Verfahren demonstriert werden.

**Beispiel 1: HSA-Einfluß auf den Estradiol-RIA mit Dextrankohletrennung**

Als erstes Beispiel wird ein sehr einfaches Assay-System mit Dextrankohletrennung gewählt. Beim Estradiol-RIA ist die Wirkung von Humanserum-Albumin (HSA) auf den Immunoassay bekannt. Es soll mit diesem Beispiel gezeigt werden, daß die zweidimensionale Eichung in der Lage ist, Estradiol (E2) bei unterschiedlichen HSA-Zugaben zu E2 auch richtig zu bestimmen.

Aufbau des zweidimensionalen Eichexperiments:

Alle Komponenten wurden in Phosphatpuffer (0.01 m, PH 7.6) unter Zusatz von 0.5 mg/ml (0.05 %) Humanserumalbumin (HSA) angesetzt. 100 µl E2-I-125-Tracer (6-E2-Jod-Histamin), 100 µl anti-E2-Antiserum, 50 µl E2-Standard bzw. bei B0 Phosphatpuffer (0.01 n, PH 7.4, 0.05 % HSA) wurden in ein Röhrchen zusammengegeben. Alle Ansätze wurden dreifach durchgeführt.

E2 wurde in den Konzentrationen 0, 0.143 und 1.42 pmol/ml als erste unabhängige Eichvariable und anti-E2-Antiserum in den Verdünnungstufen 1:5000, 1:10000, 1:20000, 1:40000, 1:80000, 1:160000, 1:320000, 1:640000 als zweite unabhängige Variable angesetzt. Als Tracer fand ein I-125-6-Histamin-E2-Konjugat in einer Konzentration von 0.05 nmol/l Verwendung.

Die Inkubation lief über 16 h bei einer Temperatur von 4° C. Danach erfolgte die Dextrankohletrennung (2.5 g/l Norit A, 0,25 g/l Dextran T70) über 15 min bei 4 °C und Zentrifugation bei 4500 U/min a 20 min. Der Überstand wurde in ein gesondertes Röhrchen decantiert. Sowohl Überstand (gebundener Tracer) als auch Kohlepräzipitat (freier Tracer) als auch Gesamttracer wurden im Gammacounter gemessen.

Die mittlere Impulsrate des gebundenen Tracers (B) aus den drei Ansätzen wurde durch die Impulsrate des Gesamttracers (T) geteilt. Das ergab als Response den B/T-Wert. Je Dreifach-ansatz wurde der Variationskoeffizient bestimmt.

Dieser Versuch wurde mit Hilfe der nichtlinearen Regression unter Verwendung der Eichfunktion

$$B/T = 0.5 \cdot (m - \sqrt{(m^2 - \frac{4 \cdot n \cdot y}{(x+p^*)}} + nsb$$

$$m = 1 + \frac{n \cdot y}{(x+p^*)} \cdot (1 + \frac{1}{K}) \tag{1}$$

x = Antigenkonzentration (hier: E2)
y = Antikörperkonzentration
n = Bindungsfaktor (Bindungsrelation Antigen zu Antikörper)
K = Bindungskonstante
nsb = unspezifische Bindung
p* = Tracerkonzentration (hier: I-125-E2)

ausgewertet. Die Auswertung brachte folgendes Ergebnis:

**Auswerteergebnis**

| | | |
|---|---|---|
| Tracerkonzentration | 0.050 nmol/l | |
| Bindungskonstante | 1.8430553 +/- | 0.1391364 |
| Bindungsfaktor | 0.0441816 +/- | 0.0020219 |
| unspezifische Bindung | 0.0351022 +/- | 0.0037625 |
| Summe der Abweichungsquadrate: 1.5809379324E-03 Modellfehler: 0.1145 | | Versuchsfehler: 0.0480 |

| WERTETABELLE | | | | | | |
|---|---|---|---|---|---|---|
| Nr. | Lauf-variable E2 nmol/l | Block-Variable anti-E2-AS 100=1:1000 | berechn. Response | experim. Response | Abweich. | VK |
| 1 | 0.000 | 10.000 | 0.6663 | 0.6610 | 0.0081 | 0.0117 |
| 2 | 0.000 | 5.000 | 0.6486 | 0.6408 | 0.0122 | 0.0320 |
| 3 | 0.000 | 2.500 | 0.6117 | 0.6217 | 0.0161 | 0.0170 |
| 4 | 0.000 | 1.250 | 0.5366 | 0.5584 | 0.0390 | 0.0106 |
| 5 | 0.000 | 0.625 | 0.4086 | 0.4341 | 0.0586 | 0.0191 |
| 6 | 0.000 | 0.313 | 0.2665 | 0.2822 | 0.0557 | 0.0256 |
| 7 | 0.000 | 0.156 | 0.1620 | 0.1687 | 0.0397 | 0.0436 |
| 8 | 0.143 | 10.000 | 0.6144 | 0.6206 | 0.0101 | 0.0118 |
| 9 | 0.143 | 5.000 | 0.5418 | 0.5256 | 0.0308 | 0.0358 |
| 10 | 0.143 | 2.500 | 0.4162 | 0.3672 | 0.1336 | 0.0209 |
| 11 | 0.143 | 1.250 | 0.2728 | 0.2341 | 0.1656 | 0.0134 |
| 12 | 0.143 | 0.625 | 0.1664 | 0.1409 | 0.1816 | 0.0370 |
| 13 | 0.143 | 0.313 | 0.1039 | 0.0902 | 0.1514 | 0.0794 |
| 14 | 0.143 | 0.156 | 0.0701 | 0.0556 | 0.2612 | 0.0516 |
| 15 | 1.429 | 10.000 | 0.2810 | 0.2928 | 0.0404 | 0.0376 |
| 16 | 1.429 | 5.000 | 0.1716 | 0.1765 | 0.0276 | 0.0033 |
| 17 | 1.429 | 2.500 | 0.1067 | 0.1089 | 0.0209 | 0.0128 |
| 18 | 1.429 | 1.250 | 0.0717 | 0.0717 | 0.0001 | 0.0105 |
| 19 | 1.429 | 0.625 | 0.0536 | 0.0481 | 0.1149 | 0.0961 |
| 20 | 1.429 | 0.313 | 0.0444 | 0.0392 | 0.1323 | 0.1230 |
| 21 | 1.429 | 0.156 | 0.0398 | 0.0346 | 0.1478 | 0.0744 |

Modellprüfung

Gewichtete lineare Regression zum Vergleich der experimentellen Response mit der berechneten Response

Ergebnis:

Relative Uebereinstimmung (b +/- db) : 1.009 +/- 0.106
Mittlere Abweichung (a +/- da): 0.000 +/- 0.017
Korrelationskoeffizient: 0.9906

Die Auswertung ist o.k., wenn b +/- db um 1 und a +/- da um 0 schwanken.
da und db sind der Vertrauensbereich von a und b

| Rekalkulationswerte der Standards | | | | |
|---|---|---|---|---|
| AK-Wert anti-E2-AS 100=1:1000 | Standard E2 nmol/l | Rekalk.Wert E2 nmol/l | Versuchsfehler nmol/l | Mittelwert E2 +/- S |
| 10.000 | 0.000 | 0.015 | 0.0218 | |
| 5.000 | 0.000 | 0.011 | 0.0278 | |
| 2.500 | 0.000 | -0.007 | 0.0071 | 0.0001 |
| 1.250 | 0.000 | -0.007 | 0.0020 | +/- 0.009 |
| 0.625 | 0.000 | -0.006 | 0.0018 | |
| 0.313 | 0.000 | -0.004 | 0.0018 | |
| 0.156 | 0.000 | -0.003 | 0.0030 | |

(fortgesetzt)

| Rekalkulationswerte der Standards | | | | |
|---|---|---|---|---|
| AK-Wert anti-E2-AS 100=1:1000 | Standard E2 nmol/l | Rekalk.Wert E2 nmol/l | Versuchsfehler nmol/l | Mittelwert E2 +/- S |
| 10.000 | 0.143 | 0.126 | 0.0196 | |
| 5.000 | 0.143 | 0.165 | 0.0263 | |
| 2.500 | 0.143 | 0.193 | 0.0089 | |
| 1.250 | 0.143 | 0.190 | 0.0046 | 0.177 |
| 0.625 | 0.143 | 0.194 | 0.0136 | +/- 0.027 |
| 0.313 | 0.143 | 0.193 | 0.0376 | |
| 0.156 | 0.143 | 0.282 | 0.0547 | |
| 10.000 | 1.429 | 1.342 | 0.0812 | |
| 5.000 | 1.429 | 1.373 | 0.0066 | |
| 2.500 | 1.429 | 1.382 | 0.0289 | 1.52 |
| 1.250 | 1.429 | 1.428 | 0.0317 | +/- 0.305 |
| 0.625 | 1.429 | 2.059 | 1.1726 | |
| 0.313 | 1.429 | 3.317 | 22.4805 | |
| 0.156 | 1.429 | -13.778 | 11.4839 | |

Rekalkulationsprüfung

Gewichtete lineare Regression zum Vergleich der Standards (x) mit den rekalkulierten Standards

Ergebnis:

Relative Uebereinstimmung (b +/- db) : 1.022 +/- 0.043
Mittlere Abweichung (a +/- da): 0.002 +/- 0.007
Korrelationskoeffizient: 0.9955

Die Übereinstimmung ist o.k., wenn b +/- db um 1 und a +/- da um 0 schwanken. da und db sind der Vertrauensbereich von a und b

Sowohl die Modellprüfung als auch die Prüfung der Rekalkulation der eingesetzten Standardkonzentrationen zeigte ein sehr gute Übereinstimmung zwischen berechneten Werten und experimentellen Werten. Bei der Rekalkulation blieben die Werte mit hohem Fehler( Werte außerhalb des Bestimmungsbereiches des Assays) auf Grund Ihres geringen Wichtungsfaktors weitgehend unberücksichtigt. Als wichtigstes Ergebnis zeigte sich jedoch, daß die Analytkonzentrationen (E2) bei unterschiedlicher Antiserumverdünnung nur statistisch um den vorgegebenen Standardwert schwankten.

Wie werden nun E2-Standardproben wiedergefunden, die statt mit 0.05 % (Eichexperiment) mit 0.1 % und mit 2 % HSA versetzt sind.

**1. Wiederfindung der E2-Standardproben in 0.1 % HSA**

Die Konzentration der mit 0.1 % HSA versetzten, bekannten E2-Konzentrationen wurden wie unbekannte Proben behandelt und bei den gleichen unterschiedlichen Antiserumverdünnungen wie im Eichexperiment angesetzt und gemessen und mittels der nach x umgeformter Eichfunktion (Gleichung 1) bestimmt.

| Wiederfindung bekannter E2-Konzentrationen bei HSA=0.1 % | | | |
|---|---|---|---|
| E2-Konz. bekannt nmol/l | AS-Verd. 100=1:1000 | E2-Konz. ermittelt nmol/l | Fehler E2-Konz. nmol/l |
| 0.0000 | 10.000 | -0.216 | 0.2511 |
| 0.0000 | 5.000 | -0.075 | 0.1124 |
| 0.0000 | 2.500 | -0.053 | 0.0524 |

(fortgesetzt)

| Wiederfindung bekannter E2-Konzentrationen bei HSA=0.1 % | | | |
|---|---|---|---|
| E2-Konz. bekannt nmol/l | AS-Verd. 100=1:1000 | E2-Konz. ermittelt nmol/l | Fehler E2-Konz. nmol/l |
| 0.0000 | 1.250 | -0.024 | 0.0221 |
| 0.0000 | 0.625 | -0.012 | 0.0104 |
| 0.0000 | 0.313 | -0.011 | 0.0072 |
| 0.0000 | 0.156 | -0.015 | 0.0054 |
| 0.1430 | 10.000 | -0.018 | 0.2004 |
| 0.1430 | 5.000 | 0.096 | 0.0847 |
| 0.1430 | 2.500 | 0.143 | 0.0478 |
| 0.1430 | 1.250 | 0.136 | 0.0307 |
| 0.1430 | 0.625 | 0.126 | 0.0280 |
| 0.1430 | 0.313 | 0.093 | 0.0252 |
| 0.1430 | 0.156 | 0.058 | 0.0169 |
| 1.4290 | 10.000 | 1.201 | 0.2346 |
| 1.4290 | 5.000 | 1.176 | 0.2152 |
| 1.4290 | 2.500 | 1.016 | 0.1508 |
| 1.4290 | 1.250 | 0.751 | 0.1374 |
| 1.4290 | 0.625 | 0.533 | 0.0848 |
| 1.4290 | 0.313 | 0.330 | 0.0509 |
| 1.4290 | 0.156 | 0.153 | 0.0451 |

Im Wiederfindeexperiment sind jedoch nur solche Proben zu berücksichtigen, deren Fehler akzeptabel sind (möglichst kleiner als 1/3-tel der Probenwerte selbst).

Das Wiederfindeexperiment kann sinnvoll in drei Teile zerlegt werden,

1. Die 0-Probe (E2=0)
2. bei geringem E2-Gehalt (E2 = 0.143 nmol/l)
3. bei hohem E2-Gehalt (E2 = 1.429 nmol/l)

| zu 1. Die 0-Probe (E2=0) | | |
|---|---|---|
| AS-Verd. 100=1:1000 | E2-Konz. ermittelt nmol/l | Fehler E2-Konz. nmol/l |
| 10.000 | -0.216 | 0.2511 |
| 5.000 | -0.075 | 0.1124 |
| 2.500 | -0.053 | 0.0524 |
| 1.250 | -0.024 | 0.0221 |
| 0.625 | -0.012 | 0.0104 |
| 0.313 | -0.011 | 0.0072 |
| 0.156 | -0.015 | 0.0054 |

Bei den 0-Proben kann auf Grund der Fehler der Bestimmungen keine signifikante Abweichung von 0 bestimmt werden.

| zu 2. bei geringem E2-Gehalt (E2 = 0.143 nmol/l) | | |
|---|---|---|
| AS-Verd. 100=1:1000 | E2-Konz. ermittelt nmol/l | Fehler E2-Konz. nmol/l |
| 10.000 | -0.018 | 0.2004 |
| 5.000 | 0.096 | 0.0847 |
| 2.500 | 0.143 | 0.0478 |
| 1.250 | 0.136 | 0.0307 |
| 0.625 | 0.126 | 0.0280 |

(fortgesetzt)

| zu 2. bei geringem E2-Gehalt (E2 = 0.143 nmol/l) | | |
|---|---|---|
| AS-Verd. 100=1:1000 | E2-Konz. ermittelt nmol/l | Fehler E2-Konz. nmol/l |
| 0.313 | 0.093 | 0.0252 |
| 0.156 | 0.058 | 0.0169 |

Die wiedergefundenen E2-Konzentrationen bei AS-Verdünnungen 10 und 5 sind nicht verwendbar, da die Bestimmungsfehler zu hoch sind. Für den Bereich AS-Verdünnung 0.156 - 2.5 finden wir mit zunehmender Antiserumverdünnung = abnehmender Antikörperkonzentration eine zunehmende systematische Abweichung vom vorgegebenen E2-Gehalt von 0.143 nmol/l.

Die Gleichung

$$x = 0.146 - 0.014 \,^\circ\, 1/y \qquad r = 99.2\,\%$$

    x : wiedergefundene E2-Konzentration in nmol/l
    y : Antikörpergehalt des Antiserums (100 = 1:1000)
    r : Korrelationskoeffizient

die durch lineare Regression gewonnen wurde, gibt diesen Sachverhalt gut wieder.

Die richtige E2-Konzentration von 0.143 wird somit beim höchst möglichen Antikörpergehalt gefunden. Die Gleichung sagt einen Höchstwert von 0.146 voraus, was mit den experimentellen Werten gut übereinstimmt. Mit abnehmendem Antikörpergehalt wird durch das zunehmende Gewicht der HSA-Konkurrenz die am Antikörper gebundenen E2-Konzentrationen immer geringer.

| zu 3. bei hohem E2-Gehalt (E2 = 1.429 nmol/l) | | |
|---|---|---|
| AS-Verd. 100=1:1000 | E2-Konz. ermittelt nmol/l | Fehler E2-Konz. nmol/l |
| 10.000 | 1.201 | 0.2346 |
| 5.000 | 1.176 | 0.2152 |
| 2.500 | 1.016 | 0.1508 |
| 1.250 | 0.751 | 0.1374 |
| 0.625 | 0.533 | 0.0848 |
| 0.313 | 0.330 | 0.0509 |
| 0.156 | 0.153 | 0.0451 |

Bei der Antiserumverdünnung 1:10.000 = 10 wird der Sättigungsbereich für diese hohe Antigenkonzentration noch nicht erreicht. Die Gleichung

$$x = 0.676 + 0.274 \cdot \ln(y) \qquad r = 98.8\,\%$$

gibt den Zusammenhang jedoch gut wieder.

Bei einer Antiserumverdünnung von 1:5000 = 20 nähern wir uns dem Bereich der Sättigungsbindung auch bei dieser hohen Antigenkonzentration. Aus der Gleichung ergibt sich dann ein Wert von 1.497 nmol/l E2, der sich nicht signifikant vom gesuchten Wert 1.429 unterscheidet.

**1. Wiederfindung der E2-Standardproben in 2 % HSA**

Mit den in 2 % HSA-Lösung angesetzten E2-Standardproben wurde in gleicher Weise, wie oben beschrieben, verfahren.

| Wiederfindung bekannter E2-Konzentrationen bei HSA = 2 % | | | |
|---|---|---|---|
| E2-Konz. bekannt nmol/l | AS-Verd. 100=1:1000 | E2-Konz. ermittelt nmol/l | Fehler E2-Konz. nmol/l |
| 0.0000 | 10.000 | 0.163 | 0.1741 |
| 0.0000 | 5.000 | 0.208 | 0.0842 |
| 0.0000 | 2.500 | 0.189 | 0.0476 |
| 0.0000 | 1.250 | 0.154 | 0.0327 |
| 0.0000 | 0.625 | 0.134 | 0.0267 |
| 0.0000 | 0.313 | 0.093 | 0.0206 |
| 0.0000 | 0.156 | 0.056 | 0.0142 |
| 0.1430 | 10.000 | 0.436 | 0.1675 |
| 0.1430 | 5.000 | 0.474 | 0.1024 |
| 0.1430 | 2.500 | 0.450 | 0.0883 |
| 0.1430 | 1.250 | 0.355 | 0.0587 |
| 0.1430 | 0.625 | 0.274 | 0.0442 |
| 0.1430 | 0.313 | 0.180 | 0.0309 |
| 0.1430 | 0.156 | 0.089 | 0.0206 |
| 1.4290 | 10.000 | 2.172 | 0.3326 |
| 1.4290 | 5.000 | 1.931 | 0.2935 |
| 1.4290 | 2.500 | 1.458 | 0.2126 |
| 1.4290 | 1.250 | 1.044 | 0.1490 |
| 1.4290 | 0.625 | 0.673 | 0.0971 |
| 1.4290 | 0.313 | 0.353 | 0.0790 |
| 1.4290 | 0.156 | 0.174 | 0.0344 |

Das Wiederfindeexperiment kann sinnvoll wieder in drei Teile zerlegt werden,

1. Die 0-Probe (E2=0)
2. bei geringem E2-Gehalt (E2 = 0.143 nmol/l)
3. bei hohem E2-Gehalt (E2 = 1.429 nmol/l)

zu 1. Die 0-Probe (E2=0)

Die durch lineare Regression aus den Werte für E2=0 gewonnene Gleichung

$$x = 0.044 \circ \ln(y) + 0.144 \qquad r = 99.3 \%$$

bestimmt bei einem Antikörpergehalt = 5 (entspricht einer Antiserumverdünnung 1:20.000) ein E2-Gehalt von ca. 0.22 nmol/l, der jedoch nicht von E2, sondern von zu E2 in HSA vorhandenen kreuzreaktiven Stoffen hervorgerufen wird.

zu 2. bei geringem E2-Gehalt (E2 = 0.143 nmol/l)

Die durch lineare Regression aus den Werte für E2 = 0.143 nmol/l gewonnene Gleichung

$$x = 0.116 \circ \ln(y) + 0.338 \qquad r = 99.1 \%$$

bestimmt bei einem Antikörpergehalt = 2.5 (entspricht einer Antiserumverdünnung 1:40.000, der noch eine Bestimmung 0.1 nmol/l E2 gestattet) ein E2-Gehalt von ca. 0.45 nmol/l. Zieht man die 0.22 nmol/l ab, die durch kreuzreaktive Substanzen verursacht wurde, wird ein E2-Gehalt von 0.23 nmol/l bestimmt, was im Fehlerbereich der Bestimmung für 0.143 nmol/l liegt.

EP 0 850 416 B1

zu 3. bei hohem E2-Gehalt (E2 = 1.429 nmol/l)

Die durch lineare Regression aus den Werte für E2 = 1.429 nmol/l gewonnene Gleichung

$$x = 0.514 \circ \ln(y) + 0.514 \qquad r = 99.1 \%$$

bestimmt bei einem Antikörpergehalt = 5 (entspricht einer Antiserumverdünnung 1:20.000) ein E2-Gehalt von ca. 1.82 nmol/l. Zieht man die 0.22 nmol/l ab, die durch kreuzreaktive Substanzen verursacht wurde, wird ein E2-Gehalt von 1.60 nmol/l bestimmt, was im Fehlerbereich der Bestimmung für 1.492 nmol/l liegt.

**Beispiel 2: Serum-Einfluß auf den Estradiol-RIA mit Dextrankohletrennung**

Anders sind die Verhältnisse, wenn Estradiol in Serum bestimmt werden soll. Um ein sinnvolles Wiederfindeexperiment dort zu konzipieren, haben wir Estradiol-Standard in männliches Poolserum, das quasi Estradiol-frei ist, als Proben angesetzt. Das Eichexperiment wurde in Pufferlösung (0.05 % HSA) ausgeführt. Statt Antiserum wurde ein Protein-G gereinigter monoklonaler Antikörper verwendet.
E2 wurde in den Konzentrationen 0 und 1.0 nmol/l als erste unabhängige Eichvariable und monoklonaler anti-E2-Antikörper in den Konzentrationen 0.25, 0.125, 0.063, 0.025, 0.013, 0.006, 0.003 und 0.001 nmol/l als zweite unabhängige Variable angesetzt. Als Tracer fand ein I-125-6-Histamin-E2-Konjugat in einer Konzentration von 0.04 nmol/l Verwendung.
Eichexperiment und Probenbestimmung wurden in gleicher Weise wie im Beispiel 1 ausgeführt.
Das Eichexperiment wurde mit Hilfe der nichtlinearen Regression unter Verwendung der Eichfunktion

$$B/T = 0.5 \cdot \left( m - \sqrt{\left(m^2 - \frac{4 \cdot n \cdot y}{(x + p^* - p_2)}\right)} + nsb$$

$$m = 1 + \frac{n \cdot y}{(x + p^* + p_2)} \cdot \left(1 + \frac{1}{K}\right) \qquad (2)$$

x = Antigenkonzentration (hier: E2)
y = Antikörperkonzentration
n = Bindungsfaktor (Bindungsrelation Antigen zu Antikörper)
K = Bindungskonstante
nsb = unspezifische Bindung
p* = Tracerkonzentration (hier: I-125-E2)
$p_2$ = Fremdsubstanz

ausgewertet. Die Auswertung brachte folgendes Ergebnis:

| | | |
|---|---|---|
| Tracerkonzentration | 0.0400000 | |
| Bindungskonstante | 0.9605006 +/- | 0.0377880 |
| Bindungsfaktor | 1.5324230 +/- | 0.0537602 |
| Unspezifische Bindung | 0.0260806 +/- | 0.0006072 |
| Fremdsubstanz | 0.0112915 +/- | 0.0026392 |
| Summe der Abweichungsquadrate: 5.9361908853E-05 Modellfehler: 0.0465 | | Versuchsfehler: 0.0745 |

| WERTETABELLE | | | | | | |
|---|---|---|---|---|---|---|
| Nr. | Estradiol nmol/l | anti-E2- MAK nmol/l | berechn.B/T | B/T | Abweich. | VK |
| 1 | 0.000 | 0.001 | 0.0620 | 0.0611 | 0.0144 | 0.0254 |
| 2 | 0.000 | 0.003 | 0.0950 | 0.0941 | 0.0100 | 0.0371 |
| 3 | 0.000 | 0.006 | 0.1780 | 0.1748 | 0.0184 | 0.0265 |
| 4 | 0.000 | 0.013 | 0.2725 | 0.2788 | 0.0227 | 0.0573 |

**11**

(fortgesetzt)

| WERTETABELLE | | | | | | |
|---|---|---|---|---|---|---|
| Nr. | Estradiol nmol/l | anti-E2- MAK nmol/l | berechn.B/T | B/T | Abweich. | VK |
| 5 | 0.000 | 0.025 | 0.3684 | 0.3864 | 0.0467 | 0.0190 |
| 6 | 0.000 | 0.063 | 0.4519 | 0.4402 | 0.0266 | 0.0303 |
| 7 | 0.000 | 0.125 | 0.4835 | 0.4754 | 0.0169 | 0.0246 |
| 8 | 0.000 | 0.250 | 0.4997 | 0.4856 | 0.0290 | 0.0175 |
| 9 | 1.000 | 0.001 | 0.0279 | 0.0281 | 0.0078 | 0.0136 |
| 10 | 1.000 | 0.003 | 0.0297 | 0.0316 | 0.0594 | 0.2432 |
| 11 | 1.000 | 0.006 | 0.0351 | 0.0332 | 0.0584 | 0.0760 |
| 12 | 1.000 | 0.013 | 0.0440 | 0.0428 | 0.0261 | 0.0612 |
| 13 | 1.000 | 0.025 | 0.0612 | 0.0595 | 0.0285 | 0.0955 |
| 14 | 1.000 | 0.063 | 0.1087 | 0.1057 | 0.0280 | 0.0160 |
| 15 | 1.000 | 0.125 | 0.1751 | 0.1813 | 0.0341 | 0.0409 |
| 16 | 1.000 | 0.250 | 0.2689 | 0.2994 | 0.1018 | 0.0270 |

Modellprüfung

Gewichtete lineare Regression zum Vergleich der experimentellen Response mit der berechneten Response

Ergebnis:

Relative Uebereinstimmung (b +/- db) : 0.998 +/- 0.067
Mittlere Abweichung (a +/- da): 0.000 +/- 0.004
Korrelationskoeffizient: 0.9976

| Rekalkulationswerte der Standard | | | |
|---|---|---|---|
| anti-E2-MAK nmol/l | Estradiol erwartet nmol/l | Estradiol rekalk. nmol/l | Fehler Rekalk. nmol/l |
| 0.001 | 0.000 | -0.009 | 0.0020 |
| 0.003 | 0.000 | 0.011 | 0.0037 |
| 0.006 | 0.000 | -0.001 | 0.0020 |
| 0.013 | 0.000 | -0.000 | 0.0059 |
| 0.025 | 0.000 | -0.007 | 0.0029 |
| 0.063 | 0.000 | 0.010 | 0.0116 |
| 0.125 | 0.000 | 0.013 | 0.0189 |
| 0.250 | 0.000 | 0.044 | 0.0271 |
| 0.001 | 1.000 | 0.706 | 0.1771 |
| 0.003 | 1.000 | 0.777 | 2.9556 |
| 0.006 | 1.000 | 1.231 | 0.7090 |
| 0.013 | 1.000 | 1.119 | 0.2214 |
| 0.025 | 1.000 | 1.054 | 0.2351 |
| 0.063 | 1.000 | 1.052 | 0.0265 |
| 0.125 | 1.000 | 0.947 | 0.0640 |
| 0.250 | 1.000 | 0.802 | 0.0488 |

Rekalkulationsprüfung

Gewichtete lineare Regression zum Vergleich der Standards (x) mit den rekalkulierten Standards

Ergebnis:

Relative Uebereinstimmung (b +/- db) : 0.992 +/- 0.041
Mittlere Abweichung (a +/- da): 0.000 +/- 0.023
Korrelationskoeffizient: 0.9796

Unabhängig von der Antikörperkonzentration werden 0 und 1 nmol/l im Rahmen des Versuchfehlers richtig wiedergefunden.

Die gleichen E2-Standards und Antikörperkonzentrationen in 0-Serum angesetzt und mit der Formel 2 und den Parametern des Eichexperiments ausgewertet, ergab folgendes Resultat:

| Estradiol zugegeben nmol/l | anti-E2-MAK nmol/l | Estradiol wiedergefunden nmol/l | Fehler der Bestimmung nmol/l |
|---|---|---|---|
| 0.0000 | 0.006 | -0.000 | 0.0038 |
| 0.0000 | 0.013 | 0.004 | 0.0047 |
| 0.0000 | 0.025 | -0.007 | 0.0088 |
| 0.0000 | 0.063 | -0.081 | 0.0208 |
| 0.0000 | 0.125 | -0.289 | 0.0615 |
| 0.0000 | 0.250 | -0.741 | 0.1440 |
| 1.0000 | 0.003 | 0.034 | 0.0119 |
| 1.0000 | 0.006 | 0.120 | 0.0149 |
| 1.0000 | 0.013 | 0.190 | 0.0181 |
| 1.0000 | 0.025 | 0.264 | 0.0272 |
| 1.0000 | 0.063 | 0.289 | 0.0268 |
| 1.0000 | 0.250 | -0.100 | 0.0768 |

Das Wiederfindeexperiment kann sinnvoll wieder in zwei Teile zerlegt werden,

1. 0-Probe (E2=0)
2. bei hohem E2-Gehalt (E2 = 1.0 nmol/l)

Für die lineare Regression fanden nur die Werte Verwendung, deren Fehler 1/3-tel des Probenwertes nicht überstiegen.

In beiden Fällen zeigte sicht deutlich, daß im Serum nicht eine Schwächung der Bindung, wie sonst üblich, zu beobachten war, sondern hier eine Verstärkung auftrat. Diese ergibt sich aus folgendem Sachverhalt:

1. Im Serum ist mit Sexualhormonbindenden Globulin, das eine hohe Affinität zum E2 hat, zu rechnen.
2. Die Trennungsmethode ist die Dextrankohletrennung, die auf der Abtrennung des freien vom gebundenen Hapten beruht. Im Überstand der Kohletrennung bleibt eine gemeinsame Fraktion zurück, die sowohl vom Antikörper als auch von dem im Serum befindlichen Sexualhormonbindenen Protein gebildet wird.

Bei anderen, auf die gebundene Fraktion gerichtete Trennungen, wie die Doppelantikörpertechnik und die Festphasentechnik, mit einem immobilisierten Antikörper, tritt dieses Phänomen nicht auf.

Mit diesem Beispiel soll jedoch gezeigt werden, daß das Verfahren auch anwendbar ist, wenn eine Bindungsverstärkung in den Proben auftritt.

zu 1. die 0-Probe (E2=0)

Die durch gewichtete lineare Regression aus den Werte für E2=0 gewonnene Gleichung

$$x = -0.381 \, ^\circ \, \ln(y) - 1.13 \qquad r = 94.5 \, \%$$

bestimmt bei einem Antikörpergehalt von 0.25 nmol/l eine negative E2-Konzentration von -0.60, die darauf zurückzuführen ist, daß die Bindung durch das Serum verstärkt wird.

zu 2. bei hohem E2-Gehalt (E2 = 1.0 nmol/l)

Die durch gewichtete lineare Regression aus den Werte für E2=1 gewonnene Gleichung

$$x = 0.093 \, ^\circ \, \ln(y) + 0.581 \qquad r = 96{,}8 \, \%$$

bestimmt bei einem Antikörpergehalt von 0.25 nmol/l eine E2-Konzentration von 0.45. Zieht man die bei E2=0 ermittelte Konzentration von -0.60 von 0.45 ab, erhalten wir 1.05 nmol/l E2, was somit die erwarteten Konzentration von 1 nmol/l im Fehler der Bestimmung richtig wiedergibt.

**Beispiel 3: Bestimmung unbekannter Seren mit einem Sandwich ELISA für Thyreoglobulin (TG-ELISA)**

Das o.g. Verfahren beschränkt sich nicht nur auf den Radioimmunoassay, sondern gilt ebenfalls für den Immunoradiometrischen Assay (IRMA) und seinen nichtradioaktiven Varianten unter Verwendung von enzymatischen Antikörperkonjugaten bzw. von Lumineszenzlabels als Nachweissubstanzen. Als Beispiel ist hier ein enzymometrisches Nachweissystem für Thyreoglobulin (TG) ausgewählt worden, daß aus einem biotinylierten Fängerantikörper, einem TG-Standard und einem Peroxidase (POD) markiertem Nachweisantikörper besteht.

Die Trennung des am Nachweisantikörper gebundenen vom freien Antigen erfolgte an Streptavidin beschichteten Mikrotiterplatten. Bei beiden Antikörpern handelte es sich um monoklonale Antikörper, die mit unterschiedlichen Bindungsdomänen des TG reagierten, so daß TG eine spezifische Brücke zwischen beiden Antikörpern bildete. Biotinylierter Fänger-Antikörper, TG und Nachweisantikörper bilden einen sogenannten Sandwich. Der Assaytyp heißt deshalb auch allgemein Sandwich-Assay.

Beschreibung des Eichexperiments

Auf die Streptavidin beschichteten Mikrotiterplatten wurde je Kavität 0.2 ml Lösungen a 1.5 µg/ml biotinylierter Antikörper gegeben und über Nacht einwirken lassen.

Der nicht an Streptavidin gebundene biotinylierte Antikörper wurde durch mehrmaliges Waschen mit verschiedenen Lösungen entfernt.

Danach wurden 50 µl TG-Standard a 0.094, 0.187, 0.375, 0.75, 1.5, 3 und 5 ng/well, 50 µl POD-markierter Antikörper a 2, 3, 4 und 5 ng/well und 100 µl Mc Ilvin-Puffer (pH=6, Zusatz von 0.5 % BSA (Rinderserumalbumin)) bei 22°C zusammengeben. Nach 4 Stunden wurden die Lösungen abgesaugt und gewaschen. Zu den an der Mikrotiterplatten-Oberfläche gebundenen POD-Antikörper wurde Ortho-Phenylendiamin (OPD) und Wasserstoffperoxid gegeben. Die Enzymreaktion wurde mit Schwefelsäurelösung gestoppt und die Extinktion in einem Mikrotiterplattenreader bei 492 nm Wellenlänge gemessen.

Das Eichexperiment wurde mit Hilfe der nichtlinearen Regression unter Verwendung der Eichfunktion

$$E = x \bullet (1 - e^{-K \bullet (y - \Delta q)}) + u \bullet y \tag{3}$$

E = Extinktion als Response
x = Antigenkonzentration (hier: TG)
y = Konzentration an POD-markiertem Antikörper
K = Bindungskonstante
$\Delta q$ = Antikörperverlust
u = unspezifische Bindung

ausgewertet.

Als Besonderheit zeigte sich, daß erst Mengen von POD-Antikörper über 1.5 ng/well eine über die unspezifische Bindung hinausgehendes Extinktion lieferten und das die unspezische Bindung selbst von der Konzentration des POD-markierten Antikörper abhing.

**Auswerteergebnis**

| Bindungskonstante ,K | 0.0956968 +/- | 0.0015155 |
|---|---|---|
| AK-Verlust, $\Delta$q | 1.4785705 +/- | 0.0133371 |
| unspezifische Bindung | 0.0227612 +/- | 0.0003522 |
| Summe der Abweichungsquadrate: 2.0760202367E-04 Modellfehler: 0.0181 | | Versuchsfehler: 0.0200 |

| WERTETABELLE | | | | | | |
|---|---|---|---|---|---|---|
| Nr. | TG ng/w | POD-AK ng/w | berechn. Extinkt. | exper. Extinkt. | Abweich. | VK |
| 1 | 0.094 | 2.000 | 0.0501 | 0.0495 | 0.0121 | 0.0143 |
| 2 | 0.187 | 2.000 | 0.0546 | 0.0575 | 0.0500 | 0.0861 |
| 3 | 0.375 | 2.000 | 0.0638 | 0.0625 | 0.0204 | 0.0792 |
| 4 | 0.750 | 2.000 | 0.0820 | 0.0825 | 0.0057 | 0.0086 |
| 5 | 1.500 | 2.000 | 0.1185 | 0.1390 | 0.1472 | 0.1000 |
| 6 | 3.000 | 2.000 | 0.1915 | 0.1950 | 0.0177 | 0.0145 |
| 7 | 5.000 | 2.000 | 0.2889 | 0.2780 | 0.0392 | 0.0203 |
| 8 | 0.187 | 3.000 | 0.0936 | 0.0960 | 0.0248 | 0.1000 |
| 9 | 0.375 | 3.000 | 0.1191 | 0.1115 | 0.0681 | 0.0444 |
| 10 | 0.750 | 3.000 | 0.1699 | 0.1615 | 0.0520 | 0.1095 |
| 11 | 1.500 | 3.000 | 0.2715 | 0.2480 | 0.0949 | 0.0513 |
| 12 | 3.000 | 3.000 | 0.4748 | 0.4175 | 0.1372 | 0.1236 |
| 13 | 5.000 | 3.000 | 0.7457 | 0.6770 | 0.1015 | 0.1000 |
| 14 | 0.094 | 4.000 | 0.1112 | 0.1090 | 0.0202 | 0.0908 |
| 15 | 0.187 | 4.000 | 0.1311 | 0.1275 | 0.0285 | 0.0499 |
| 16 | 0.375 | 4.000 | 0.1714 | 0.1620 | 0.0583 | 0.0786 |
| 17 | 0.750 | 4.000 | 0.2518 | 0.2400 | 0.0493 | 0.0295 |
| 18 | 1.500 | 4.000 | 0.4126 | 0.4255 | 0.0303 | 0.0316 |
| 19 | 3.000 | 4.000 | 0.7342 | 0.7335 | 0.0010 | 0.0087 |
| 20 | 5.000 | 4.000 | 1.1630 | 1.0510 | 0.1066 | 0.1319 |
| 21 | 0.094 | 5.000 | 0.1407 | 0.1425 | 0.0126 | 0.0844 |
| 22 | 0.187 | 5.000 | 0.1673 | 0.1895 | 0.1171 | 0.0410 |
| 23 | 0.375 | 5.000 | 0.2211 | 0.2195 | 0.0072 | 0.0354 |
| 24 | 0.750 | 5.000 | 0.3284 | 0.3370 | 0.0256 | 0.0210 |
| 25 | 1.500 | 5.000 | 0.5429 | 0.5680 | 0.0441 | 0.1096 |
| 26 | 3.000 | 5.000 | 0.9721 | 1.0400 | 0.0653 | 0.0367 |

Modellprüfung

Gewichtete lineare Regression zum Vergleich der experimentellen Response mit der berechneten Response

Ergebnis:

Relative Uebereinstimmung (b +/- db) : 0.997 +/- 0.028
Mittlere Abweichung (a +/- da): 0.000 +/- 0.003
Korrelationskoeffizient: 0.9982

| Rekalkulationswerte der Standard | | | |
|---|---|---|---|
| POD-AK ng/w | TG-Standard ng/w | TG rekalk. ng/w | Fehler d.Bestimmung ng/w |
| 2.000 | 0.094 | 0.082 | 0.0145 |
| 4.000 | 0.094 | 0.084 | 0.0462 |
| 5.000 | 0.094 | 0.100 | 0.0420 |
| 2.000 | 0.187 | 0.246 | 0.1017 |
| 3.000 | 0.187 | 0.205 | 0.0709 |
| 4.000 | 0.187 | 0.170 | 0.0297 |
| 5.000 | 0.187 | 0.265 | 0.0272 |
| 2.000 | 0.375 | 0.349 | 0.1017 |
| 3.000 | 0.375 | 0.319 | 0.0365 |
| 4.000 | 0.375 | 0.331 | 0.0594 |
| 5.000 | 0.375 | 0.369 | 0.0272 |
| 2.000 | 0.750 | 0.760 | 0.0146 |
| 3.000 | 0.750 | 0.688 | 0.1305 |
| 4.000 | 0.750 | 0.695 | 0.0330 |
| 5.000 | 0.750 | 0.780 | 0.0247 |
| 2.000 | 1.500 | 1.920 | 0.2856 |
| 3.000 | 1.500 | 1.326 | 0.0939 |
| 4.000 | 1.500 | 1.560 | 0.0627 |
| 5.000 | 1.500 | 1.588 | 0.2176 |
| 2.000 | 3.000 | 3.071 | 0.0581 |
| 3.000 | 3.000 | 2.577 | 0.3809 |
| 4.000 | 3.000 | 2.997 | 0.0298 |
| 5.000 | 3.000 | 3.237 | 0.1334 |
| 2.000 | 5.000 | 4.776 | 0.1159 |
| 3.000 | 5.000 | 4.493 | 0.4997 |
| 4.000 | 5.000 | 4.478 | 0.6466 |

Rekalkulationsprüfung

Gewichtete lineare Regression zum Vergleich der Standards (x) mit den rekalkulierten Standards

Ergebnis:

Relative Uebereinstimmung (b +/- db) : 1.002 +/- 0.001
Mittlere Abweichung (a +/- da): -0.007 +/- 0.002
Korrelationskoeffizient: 0.9968

Die Rekalkulation zeigt eine nur im Rahmen des Versuchsfehlers liegende Schwankung der TG-Standardkonzentrationen bei unterschiedlichen Antikörperkonzentrationen.

Probenbestimmung

Bei gleichen Antikörperkonzentrationen und gleichen Versuchsbedingungen wie im Eichexperiment wurden Serumproben an Stelle von TG-Standards angesetzt und und im Mikrotiterplatten-Reader gemessen. Die gemessenen Werte wurden mittels Gleichung 3 in TG-Konzentrationen umgerechnet.

Die so bestimmten TG-Konzentrationen ergaben für die einzelnen Proben folgendes Resultat:

| Proben bezeichnung | POD-MAK ng/w | TG-Gehalt ng/w | TG-Fehler ng/w |
|---|---|---|---|
| 1v0.5 | 2.00 | 4.56 | 0.09 |
| 1v0.5 | 3.00 | 3.08 | 0.19 |
| 1v0.5 | 4.00 | 2.61 | 0.11 |
| 1v0.5 | 5.00 | 2.53 | 0.19 |
| | | | |
| 1v1 | 2.00 | 6.80 | 0.38 |
| 1v1 | 3.00 | 4.54 | 0.04 |
| 1v1 | 4.00 | 4.00 | 0.43 |
| 1v1 | 5.00 | 3.44 | 0.13 |

| Probenbezeichnung | POD-MAK ng/w | TG-Gehalt ng/w | TG-Fehler ng/w |
|---|---|---|---|
| 2v0.5 | 2.00 | 7.02 | 0.39 |
| 2v0.5 | 3.00 | 4.75 | 0.21 |
| 2v0.5 | 4.00 | 4.02 | 0.14 |
| 2v0.5 | 5.00 | 3.84 | 0.40 |
| | | | |
| 2v1 | 2.00 | 8.08 | 0.10 |
| 2v1 | 3.00 | 5.39 | 0.05 |
| 2v1 | 4.00 | 4.91 | 0.18 |
| 2v1 | 5.00 | 4.60 | 0.58 |
| | | | |
| 3v1 | 2.00 | 1.67 | 0.13 |
| 3v1 | 3.00 | 1.17 | 0.04 |
| 3v1 | 4.00 | 1.00 | 0.08 |
| 3v1 | 5.00 | 1.03 | 0.05 |
| | | | |
| 4v1 | 2.00 | 4.60 | 0.35 |
| 4v1 | 3.00 | 3.02 | 0.03 |
| 4v1 | 4.00 | 2.70 | 0.18 |
| 4v1 | 5.00 | 2.55 | 0.02 |

Alle aufgeführten Proben waren bei 4 Konzentrationen an POD-markierten Antikörper (2,3,4 und 5 ng/well) in je 2 Replikaten angesetzt und bestimmt worden. In allen Proben zeigte sich die gleiche Tendenz; die Probenwerte stiegen mit abnehmender Antikörperkonzentration.

Betrachtet man sich jede Probe einzeln, wird folgende Antikörperabhängigkeit deutlich:

**1. Probe 1v1**

| POD-MAK ng/w | TG-Gehalt ng/w | TG-Fehler ng/w |
|---|---|---|
| 2.00 | 6.80 | 0.38 |
| 3.00 | 4.54 | 0.04 |
| 4.00 | 4.00 | 0.43 |
| 5.00 | 3.44 | 0.13 |

Die gewichtete, lineare Regression zeigt folgende Abhängigkeit:

$$x = 1.525 + 9.078/y \qquad r = 92.9 \text{ \%}$$

x : Antigenkonzentration (hier: TG)

y : Konzentration an POD-markierten Antikörper

r : Korrelationskoeffizient

Da in der Serumprobe mit dem Antikörper um das TG konkurrierende Substanzen enthalten sind, bringt die kleinste, noch sinnvoll bestimmbare Konzentration an Nachweis-Antikörper im Sandwich-Assay im Vergleich zum Eichexperiment die richtige TG-Konzentration. Eine Antikörperkonzentration von 1.7 ng/well erscheint nach den Versuchsparametern als ein sinnvoller Wert. Diesen Wert in die Gleichung eingesetzt, ergibt 6.9 ng/well.

## 2. Probe 1v0.5

| POD-MAK ng/w | TG-Gehalt ng/w | TG-Fehler ng/w |
|---|---|---|
| 2.00 | 4.56 | 0.09 |
| 3.00 | 3.08 | 0.19 |
| 4.00 | 2.61 | 0.11 |
| 5.00 | 2.53 | 0.19 |

Ergebnis der gewichteten, linearen Regression:

$$x = 0.789 + 7.492/y \qquad r = 98.0\ \%$$

Die annähernd richtige Konzentration der Probe bei 1.7 ng/w Antikörperkonzentration ergab sich aus der Gleichung mit 5.2 ng/w TG.

## 3. Probe 2v1

| PDD-MAK ng/w | TG-Gehalt ng/w | TG-Fehler ng/w |
|---|---|---|
| 2.00 | 8.08 | 0.10 |
| 3.00 | 5.39 | 0.05 |
| 4.00 | 4.91 | 0.18 |
| 5.00 | 4.60 | 0.58 |

Ergebnis der gewichteten, linearen Regression:

$$x = 0.506 + 14.89/y \qquad r = 94.6\ \%$$

Die annähernd richtige Konzentration der Probe bei 1.7 ng/w Antikörperkonzentration ergab sich aus der Gleichung mit 9.3 ng/w TG.

## 4. Probe 2v0.5

| PDD-MAK ng/w | TG-Gehalt ng/w | TG-Fehler ng/w |
|---|---|---|
| 2.00 | 7.02 | 0.39 |
| 3.00 | 4.75 | 0.21 |
| 4.00 | 4.02 | 0.14 |

(fortgesetzt)

| PDD-MAK ng/w | TG-Gehalt ng/w | TG-Fehler ng/w |
|:---:|:---:|:---:|
| 5.00 | 3.84 | 0.40 |

Ergebnis der gewichteten, linearen Regression:

$$x = 1.30 + 10.89/y \qquad r = 98{,}2\ \%$$

Die annähernd richtige Konzentration der Probe bei 1.7 ng/w Antikörperkonzentration ergab sich aus der Gleichung mit 7.7 ng/w TG.

**5. Probe 3v1**

| PDD-MAK ng/w | TG-Gehalt ng/w | TG-Fehler ng/w |
|:---:|:---:|:---:|
| 2.00 | 1.67 | 0.13 |
| 3.00 | 1.17 | 0.04 |
| 4.00 | 1.00 | 0.08 |
| 5.00 | 1.03 | 0.05 |

Ergebnis der gewichteten, linearen Regression:

$$x = 0.64 + 1.68/y \qquad r = 90{,}7\ \%$$

Die annähernd richtige Konzentration der Probe bei 1.7 ng/w Antikörperkonzentration ergab sich aus der Gleichung mit 1.6 ng/w TG.

**6. Probe 4v1**

| PDD-MAK ng/w | TG-Gehalt ng/w | TG-Fehler ng/w |
|:---:|:---:|:---:|
| 2.00 | 4.60 | 0.35 |
| 3.00 | 3.02 | 0.03 |
| 4.00 | 2.70 | 0.18 |
| 5.00 | 2.55 | 0.02 |

Ergebnis der gewichteten, linearen Regression:

$$x = 1.81 + 3.68/y \qquad r = 98{,}0\ \%$$

Die annähernd richtige Konzentration der Probe bei 1.7 ng/w Antikörperkonzentration ergab sich aus der Gleichung mit 4.1 ng/w TG.

**Patentansprüche**

**1.** Verfahren zur zweidimensionalen Bestimmung von Proben in Immunoassays dadurch gekennzeichnet,

- daß die Eichung eines Immunoassays zweidimensional durchgeführt wird, wobei neben der Standardkonzentration des Antigens als weitere unabhängige Variable die Antikörperkonzentration herangezogen wird

- unbekannte Proben bei mehreren Antikörperkonzentrationen, die den Antikörperkonzentrationen bei der Eichung entsprechen, gemessen werden und anschließend ein Vergleich mit den Eichkurven erfolgt, bei denen die Änderung der ermittelten Antigenkonzentration in Abhängigkeit von der Antikörperkonzentration der Proben genutzt wird, um die tatsächliche in der Probe vorhandene Konzentration des Antigens zu bestimmen.

**2.** Verfahren nach Anspruch 1,
dadurch gekennzeichnet,
daß die gleiche Standardkurve bei 2 bis 7 verschiedenen Antikörperkonzentrationen aufgenommen wird, wobei die Eichung so erfolgt, daß bei der Rekalkulation der Standardkonzentrationen graphisch oder mittels Eichfunktion die vorgegebenen Standardkonzentrationen bei unterschiedlichen Antikörperkonzentrationen gleich sind.

**Claims**

**1.** Process for two-dimensional determination of samples in immunoassays wherein

the calibration of an immunoassay is done two-dimensionally, with not only the standard concentration of the antigen, but also the antibody concentration being used as a further independent variable,

unknown samples are measured at a number of antibody concentrations, which correspond to the antibody concentrations upon calibration, there subsequently being a comparison with the calibration curves in which the alteration of the antigen concentration as a function of the antibody concentration of the samples is used in order to determine the concentration of the antigen actually existing in the sample.

**2.** Process according to claim 1,
wherein
the same standard curve is recorded in 2 to 7 different antibody concentrations, with the calibration being done in such a way that in the recalculation of the standard concentrations, graphically or by means of the calibration function, the required standard concentrations are identical with various antibody concentrations.

**Revendications**

**1.** Procédé permettant l'analyse bidimensionnelle d'échantillons au cours d'immuno-essais
se caractérisant par le fait que

- l'étalonnage d'un immuno-essai se fait à deux dimensions, tout en mettant à contribution en plus de la concentration standard de l'antigène la concentration en anticorps en tant que variable indépendante supplémentaire;

- des échantillons inconnus en présence de plusieurs concentrations en anticorps, qui correspondent aux concentrations en anticorps relevées lors de l'étalonnage, sont mesurés et qu'ensuite une comparaison avec les courbes d'étalonnage a lieu, courbes sur lesquelles la modification de la concentration en antigène déterminée est mise à profit en fonction de la concentration en anticorps des échantillons afin d'évaluer la concentration en antigène existant réellement dans l'échantillon.

**2.** Procédé selon la revendication 1,
se caractérisant par le fait que
la même courbe standard est enregistrée pour 2 à 7 concentrations en anticorps différentes, l'étalonnage devant se faire de sorte que lors du recalcul des concentrations standards, sous forme de graphique ou au moyen de la fonction étalon, les concentrations standards prédéterminées soient les mêmes pour des concentrations en anticorps différentes.